# EUROPÄISCHE PATENTANMELDUNG

(11) **EP 3 895 738 A1**
(43) Veröffentlichungstag der Anmeldung: **20.10.2021**
(21) Anmeldenummer: 20169750.5
(22) Anmeldetag: 16.04.2020
(51) Int. Cl.: A61L 2/14, C02F 1/46, H01J 37/32, H05H 1/00, A61B 18/04

(54) **EINRICHTUNG UND VERFAHREN ZUR PLASMAAKTIVIERUNG EINER FLÜSSIGKEIT**

(71) Anmelder: Erbe Elektromedizin GmbH, 72072 Tübingen (DE)
(72) Erfinder: Brunecker, Kristin, 72108 Rottenburg (DE); Neugebauer, Alexander, 72116 Moessingen (DE)
(74) Vertreter: Rüger Abel Patentanwälte PartGmbB

(57) **Zusammenfassung**

Mit der Erfindung wird eine Einrichtung (10) zur Erzeugung plasmaaktivierter Flüssigkeit (F) mit definierten Eigenschaften bereitgestellt. Die Einrichtung (10) umfasst eine mit einem Plasma-Applikator (29) versehene Plasmaapplikationseinrichtung (25), in der eine Flüssigkeit (F) mit einem Gas-Plasma (30) in Berührung gebracht wird. Eine Sensoreinrichtung (36) dient der Analyse der Zusammensetzung der plasmabehandelten Flüssigkeit (F) wenigstens hinsichtlich einer durch die Plasmabehandlung erzeugten Spezies. Anhand der von der Sensoreinrichtung erfassten Konzentration einer oder mehrerer Spezies können die Behandlungsparameter der Flüssigkeit in dem Plasma-Applikator (29) eingestellt oder verändert werden. Damit gelingt die Bereitstellung einer plasmabehandelten Flüssigkeit (F) mit definierten Eigenschaften zur Behandlung eines Patienten.

## Beschreibung

Die Erfindung betrifft eine Vorrichtung und ein Verfahren zur Plasmaaktivierung einer Flüssigkeit zur Anwendung an einem Patienten.

Durch Einwirkung eines Plasmas auf eine Flüssigkeit, wie beispielsweise eine NaCl-Lösung, eine Ringer-Laktat-Lösung oder eine andere, kann diese Flüssigkeit in einen plasmaaktivierten Zustand überführt werden. Die Flüssigkeit enthält dann reaktive Spezies, zum Beispiel Radikale mit erhöhtem Redox-Potential sowie Substanzen wie Peroxide, Peroxinitrite, Nitrate, Nitrite, Stickoxide (Sauerstoff- bzw. Stickstoff-zentrierte reaktive Spezies), die eine medizinische Wirksamkeit besitzen. Derart aktivierte Flüssigkeiten können für eine indirekte Plasmabehandlung verwendet werden. Die Wirkung auf das zu behandelnde Gewebe kann eine desinfizierende Wirkung sein. Mikrobielle und bakterielle Organismen können durch die Flüssigkeit inaktiviert werden. Außerdem kann die Behandlung darauf beruhen, dass die plasmaaktivierte Flüssigkeit auf pathogene und gesunde Zellen unterschiedlich wirkt. Die unterschiedliche Wirkung beruht auf den in der plasmaaktivierten Flüssigkeit enthaltenen Spezies.

Zur Erzeugung plasmaaktivierter Flüssigkeiten schlägt die WO 2015/123720 A1 die Einwirkung eines Plasma auf eine Gel-Oberfläche vor. Dabei wird insbesondere auf nicht thermische sogenannte kalte Plasmas verwiesen, die eine hochaktive Mischung aus Sauerstoff-, Stickstoff- und Hydrogenradikalen, Ionen, Elektronen, Photonen und ultravioletter Strahlung aufweisen.

Die US 2012/0111721 A1 und die WO 2007/117634 A2 schlagen eine Einrichtung zur Plasmabehandlung einer Flüssigkeit vor. Die Plasmabehandlung findet in einem rotationssymmetrischen Gefäß statt, an dessen Wandung ein dünner Flüssigkeitsfilm entlang strömt. Die Entladung findet dabei zwischen zwei Graphitelektroden statt, wobei die entstehende Strahlung auf die Flüssigkeit einwirkt.

Die EP 1 702 678 A1 schlägt die Behandlung eines Flüssigkeitsfilms mit Elektronenstrahlen in einem Vakuumgefäß vor. Alternativ wird in dieser Druckschrift die Behandlung des Flüssigkeitsfilms mit einer UV-Quelle in Betracht gezogen, die anstelle der Elektronenstrahlquelle oder zusätzlich zu dieser vorgesehen ist.

Die EP 2 937 103 A1 schlägt die Aktivierung eines Wirkmediums, beispielsweise einer Wundauflage, durch Plasma vor. Die Wundauflage kann hierzu ein plasmaaktivierbares Gel, eine plasmaaktivierbare Flüssigkeit oder eine plasmaaktivierbare fluidgetränkte Trägerschicht aufweisen. Durch die Plasmabehandlung des Wirkmediums wird in diesem ein Wirkstoff erzeugt oder freigesetzt. Als Wirkmedien sind wasserbasierte Flüssigkeiten, Kochsalzlösungen und davon abgeleitete Gele vorgesehen. Zur Plasmabehandlung ist ein Plasmagenerator vorgesehen, der zum Beispiel ein kaltes Plasma bei Atmosphärendruck erzeugt. Dieses Plasma wird flächig auf das Wirkmedium appliziert. Nach Aktivierung kann die Wundauflage am Patienten angebracht werden.

Die US 2019/0110933 A1 und die WO 2017/167748 A1 schlagen eine Wundbehandlung durch kombinierte Plasma und Unterdruckanwendung vor. Dazu ist eine Vakuumtherapieeinrichtung mit einer antimikrobakteriellen und wundheilenden atmosphärischen Plasmaquelle verbunden, wobei eine Sensoreinrichtung zur Erfassung der Wundheilung vorgesehen sein kann. Dazu kann das Sensorsystem auf der Körperoberfläche des Patienten wenigstens einen physikalischen Parameter erfassen. Anhand dieses erfassten Parameters wird der Zustand der Wunde beurteilt. Erfasste Parameter können insbesondere die Temperatur, der Druck, die Feuchtigkeit und/oder pH-Wert sein.

Aus der WO 2017/074979 A1 ist die Problematik der Verschlechterung der medizinischen Wirkung plasmaaktivierter Flüssigkeiten bei längerer Lagerung bekannt. Um solche plasmaaktivierten Flüssigkeiten länger lagerfähig zu halten, wird vorgeschlagen, zur Plasmaaktivierung Flüssigkeiten heranzuziehen, deren Gehalt an Cystein, Methionin, Phenylalanin und Phenolrot reduziert ist und der 3-Nitro-L-Tyrosin zugesetzt ist.

Die US 2014/0322096 A1 und WO 2014/055812 A1 beschreiben ein Desinfektionssystem, insbesondere zur Handdesinfektion von Chirurgen. Dazu ist ein Reaktionsgefäß vorgesehen, in dem zwischen zwei isolierten Elektroden eine Barriereentladung stattfindet. Durch das dadurch entstehende Plasma wird eine zerstäubte Waschflüssigkeit geleitet, die sich in dem Gefäß am Boden sammelt und dann über Düsen zur Handdesinfektion ausgegeben werden kann.

Die US 2015/0306258 A1 beschreibt ein Verfahren zur Sterilisierung von Hornhautgewebe vor deren Transplantation. Dazu wird das Gewebe einem plasmaaktivierten Fluid ausgesetzt. Zur Plasmaaktivierung wird das Fluid in zerstäubter Form vor dem Aufbringen auf die Hornhaut als Sprühkegel durch eine Kaltplasmaentladung hindurch geleitet.

Die WO 2017/091534 A1 beschreibt Verfahren und System zum Abtöten oder Deaktivieren von Sporen durch Anwendung einer Flüssigkeit auf die zu sterilisierende Oberfläche, wobei die Flüssigkeit ein Additiv enthält, um die Plasmaaktivierung wenigstens über eine Zeitspanne von 30s zu halten. Das Additiv kann beispielsweise ein Nitrit, ein bioaktives Öl, eine Säure, ein Übergangsmetall oder ein Enzym sein.

Dagegen schlagen die WO 2014/145570 A1 und die WO 2014/152256 A1 die direkte Einwirkung eines Plasmas auf eine Wasseroberfläche vor, wodurch in dem Wasser Radikale erzeugt werden. Das plasmaaktivierte Wasser kann zur Oberflächendesinfektion angewandt werden.

Die WO 2017/083323 A1 befasst sich ebenfalls mit der Erzeugung einer plasmaaktivierten Flüssigkeit. Dazu ist eine Vorrichtung vorgesehen, in der ein aus Gas und Flüssigkeit bestehendes Aerosol an einem oder mehreren Plasmageneratoren vorbeigeführt wird, um das Gas und/oder die Flüssigkeit zu aktivieren. Zur Trennung des Gases und der Flüssigkeit nach der Plasmabehandlung ist eine Abscheideeinrichtung vorgesehen. Die Flüssigkeit kann zu Desinfektionszwecken verwendet werden. Zur Plasmaerzeugung dient eine Barriereentladung.

Die WO 2018/089577 A1 befasst sich mit Systemen und Verfahren zur Plasmaaktivierung von Flüssigkeiten, wobei die Aktivierung besonders hohe Konzentrationen erreichen soll und wobei große Flüssigkeitsmengen aktiviert werden sollen. Dazu wird eine Vorrichtung vorgesehen, die die Flüssigkeit in eine dünne Schicht überführt, wobei das Plasma nahe der dünnen Flüssigkeitsschicht erzeugt wird.

Aus der US 2019/0279849 A1 ist die Plasmaaktivierung einer Flüssigkeit einer Entladung bekannt, bei der der Stromfluss von einer Elektrode zur Flüssigkeit führt. Es werden Abstände von 6 bis 10 mm vorgeschlagen. Außerdem ist eine Lichtaufnahmeeinrichtung vorgesehen, um im Rahmen der optischen Emissionsspektroskopie typische Linien erzeugter Radikale oder Ionen zu erfassen.

Ebenfalls mit direkter Einwirkung einer elektrischen Entladung auf eine Flüssigkeit und Stromfluss durch diese arbeitet die CN 109121278 A**.** Die Entladung kann auch eine elektrische Barriereentladung sein.

Die WO 2017/192618 A1 beschreibt eine Vorrichtung und ein Verfahren zur Erzeugung eines plasmaaktivierten wässrigen Chemotherapeutikums. Dazu ist ein Entladungsgefäß vorgesehen, in dem mindestens eine hohle Nadelelektrode angeordnet ist, wobei das Gefäß innen elektrisch isolierend ausgekleidet ist. Die Elektroden sind an einer rotierenden Hohlwelle befestigt, in die Luft eingeleitet wird. Zwischen den Elektroden und der Gefäßwandung bildet sich eine Barriereentladung aus. In das Gefäß eingeleitete Flüssigkeit gelangt in innigen Kontakt mit der Barriereentladung und wird am unteren Ende des Gefäßes aktiviert ausgegeben.

Mit den genannten Verfahren lassen sich plasmaaktivierte wässrige Flüssigkeiten herstellen. Es ist auch bekannt, dass solche aktivierten Flüssigkeiten zumindest begrenzt lagerbar sind. Die aktivierten Flüssigkeiten wirken dabei breitbandig, wobei sich ihre Wirksamkeit mit der Zeit verändert. Dies kann einen Unsicherheitsfaktor bei der Anwendung plasmaaktivierter Flüssigkeiten darstellen.

Davon ausgehend ist es Aufgabe der Erfindung, die Reproduzierbarkeit des Behandlungserfolgs bei medizinischer Anwendung von plasmaaktivierten Flüssigkeiten zu verbessern.

Die erfindungsgemäße Einrichtung zur Versorgung eines medizinischen Instruments mit einer plasmaaktivierten Flüssigkeit weist einen Plasmagenerator und eine Plasmaapplikationseinrichtung auf, mittels derer das von dem Plasmagenerator erzeugte Plasma mit der Flüssigkeit in Kontakt gebracht wird. Die Flüssigkeit kann über einen Ausgang der Plasmaapplikationseinrichtung fortwährend oder portionsweise ausgeleitet und einem Instrument zugeleitet werden. Dabei kann die Flüssigkeit unmittelbar an das Instrument gegeben oder über einen Zeitraum in einem Vorratsgefäß zwischengelagert werden.

Erfindungsgemäß ist eine Sensoreinrichtung zur Erfassung wenigstens eines chemischen oder physikalischen Parameters der Flüssigkeit während und/oder nach der Plasmaexposition vorgesehen. Der von der Sensoreinrichtung erfasste Parameter wird von einer Steuereinrichtung zur Steuerung der Eigenschaften der an das Instrument abgegebenen plasmaaktivierten Flüssigkeit herangezogen. Die Steuereinrichtung weist einen Eingang auf, der mit der Sensoreinrichtung verbunden ist. Ausgangsseitig kann die Steuereinrichtung zum Beispiel mit dem Plasmagenerator verbunden sein, um Parameter der Plasmaentladung zu beeinflussen. Zusätzlich oder alternativ kann die Steuereinrichtung mit der Plasmaapplikationseinrichtung, Pumpen, Ventilen oder dergleichen Regelorganen verbunden sein, um Parameter der Plasmabehandlung zu beeinflussen. So beispielsweise die Verweildauer der Flüssigkeit im oder am Plasma. Wird die Flüssigkeit in kompakter Form, als dünner Film oder als Tröpfchen mit dem Plasma in Kontakt gebracht, kann die Steuereinrichtung die Strömungsgeschwindigkeit der Flüssigkeit, die Tröpfchengröße der Flüssigkeit, die Schichtdicke der Flüssigkeit und dergleichen mehr beeinflussen. Zusätzlich oder alternativ kann die Steuereinrichtung mit Steuerorganen eines Vorratsgefäßes, wie beispielsweise Ventilen oder Pumpen, verbunden sein, um die Verweildauer der aktivierten Flüssigkeit im Vorratsgefäß zu beeinflussen.

Der Plasmagenerator weist vorzugsweise einen Gaskanal und wenigstens eine mit Gas aus dem Gaskanal in Berührung stehende Elektrode auf. Die Elektrode kann eine isolierte Elektrode sein, um eine Barriereentladung zu speisen. Es kann sich auch um eine Elektrode mit elektrisch leitender Oberfläche handeln, um einen direkten Übergang von Elektronen aus der Elektrode in das Plasma zu ermöglichen. Der Gaskanal kann mit einem Gas, insbesondere einem Inertgas wie beispielsweise Argon, beaufschlagt sein. Es kann so an der Elektrode eine Ionisierung des Gases und somit die Erzeugung eines Plasmas, insbesondere eines Plasma-strahls stattfinden, der die Flüssigkeit berührt. Die Flüssigkeit kann mit einer Elektrode in elektrischem Kontakt stehen. Vorzugsweise handelt es sich dabei um eine nicht isolierte Elektrode. Sie weist dann eine elektrisch leitfähige Oberfläche auf, so dass ein Übertritt von Elektronen von der Flüssigkeit in die Elektrode und von der Elektrode in die Flüssigkeit möglich ist. Die Flüssigkeitsaktivierung durch Plasma erfolgt vorzugsweise durch Einwirkung des Plasmastrahls auf die Flüssigkeit. Die Flüssigkeit kann dabei als kompakter Körper mit einer zum Beispiel horizontalen Flüssigkeitsoberfläche bereitgehalten werden. Der Plasmastrahl kann auch in die Flüssigkeit eingeleitet werden, sodass Teile des Plasmas als Blasen in der Flüssigkeit aufsteigen. Außerdem kann zerstäubte Flüssigkeit in den Argon-Plasma-Strahl eingeleitet werden. Es ist aber auch möglich, anstelle des Argon-Plasma-Strahls andere Arten von Plasma insbesondere Luftplasma zu verwenden. Das Plasma kann ein Kaltplasma oder auch ein Plasma sein, dessen Gastemperatur "warm" ist, d.h., dessen Gastemperatur oberhalb der Körpertemperatur des Menschen liegt.

Insbesondere eignet sich zur Plasmaaktivierung der Flüssigkeit ein Plasmagenerator mit nicht isolierter Elektrode, die einen Stromfluss durch die Flüssigkeit ermöglicht.

Durch die Plasmabehandlung werden in der Flüssigkeit Spezies, d.h. chemische Verbindungen mit unterschiedlicher Lebensdauer und Reaktivität erzeugt, die insgesamt als plasmaerzeugte Substanzen bezeichnet werden. Solche plasmaerzeugten Substanzen können Hydroniumionen, Hydroxydionen, Wasserstoffperoxid und/oder Nitritionen und/oder Nitrationen und/oder Peroxynitrit und/oder Singulettsauerstoff und/oder Ozon und/oder Sauerstoff und/oder Superoxid-Radikal-Ionen und/oder Hydroxylradikale und/oder Hydroperoxylradikale und/oder Stickoxide wie Stickstoffmonoxid und Stickstoffdioxid sein. Die zur Erfassung solcher Spezies vorgesehene Sensoreinrichtung kann eine Sensoreinrichtung sein, die zur optischen Spektroskopie beispielsweise zur Absorptionsspektroskopie für Ultraviolett-Licht, sichtbares Licht oder Infrarot-Licht geeignet ist. Zusätzlich oder alternativ kann die Sensoreinrichtung eine zur Erfassung der Phosphoreszenz, insbesondere der Nahinfrarot-Phosphoreszenz, geeignete Einrichtung sein. Die Sensoreinrichtung kann zusätzlich oder alternativ eine zur Durchführung der optischen Emissionsspektroskopie für UV-Licht, sichtbares Licht und/oder nahes Infrarotlicht sein. Zusätzlich oder alternativ kann die Sensoreinrichtung eine Elektronenspinresonanz-Spektroskopie-Einrichtung sein.

Weitere Einzelheiten vorteilhafter Ausführungsformen der Erfindung sind Gegenstand der Beschreibung oder der Zeichnung. Es zeigen:
Figur 1 ein Blockbild zur Veranschaulichung struktureller und funktioneller Elemente der erfindungsgemäßen Einrichtung zur Versorgung eines medizinischen Elements mit einer plasmaaktivierten Flüssigkeit,
Figur 2 eine Plasmaapplikationseinrichtung zur Erzeugung plasmabehandelter Flüssigkeiten.

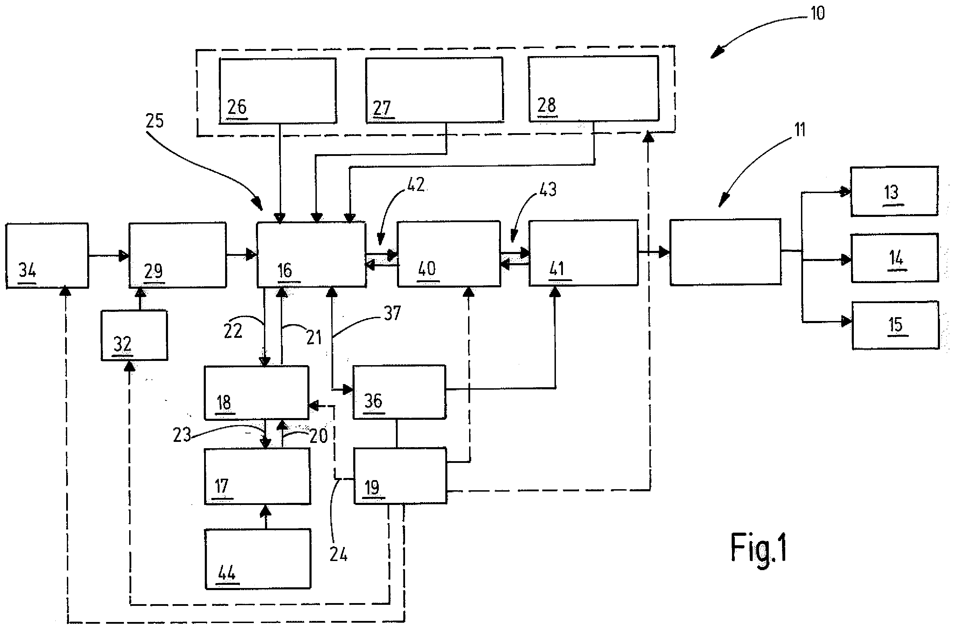

In Figur 1 ist eine Einrichtung 10 zur Versorgung eines medizinischen Instruments 11 mit plasmaaktivierter Flüssigkeit veranschaulicht, mit dem wenigstens eines von verschiedenen Behandlungsverfahren durchgeführt werden kann. Behandlungsverfahren sind in Figur 1 durch Funktionsblöcke veranschaulicht, wobei Block 13 für das Unterspritzen von Gewebe mit plasmaaktivierter Flüssigkeit steht. Block 14 steht für die abwechselnde Behandlung von Gewebe mit Plasma, z.B. Argonplasma, und/oder mit HF-Strom sowie plasmaaktivierter Flüssigkeit. Block 15 steht für das Benetzen oder Besprühen von Gewebe mit plasmaaktivierter Flüssigkeit.

Unter einem Instrument 11 versteht sich jedes Instrument das wenigstens eine der genannten in den Blöcken 13 bis 15 dargestellten Verfahren ausüben kann sowie auch andere Instrumente zur anderweitigen Applikation von plasmaaktivierter Flüssigkeit an einem Patienten oder an biologischem Gewebe.

Die Einrichtung 10 weist ein Behandlungsgefäß 16 auf, in dem eine Flüssigkeit, beispielsweise Natriumchloridlösung, Ringer-Lösung, Ringer-Laktat-Lösung oder eine andere am Patienten anwendbare Flüssigkeit mit Plasma zu behandeln ist. Die Flüssigkeit entstammt einem Vorratsgefäß 17, das über eine steuerbare Pumpe 18 mit dem Behandlungsgefäß 16 verbunden ist. Die Pumpe 18 dient dazu, gesteuert von einer Steuereinrichtung 19 Flüssigkeit aus dem Vorratsgefäß 17 in das Behandlungsgefäß 16 zu pumpen, wie Pfeile 20, 21 andeuten. Zusätzlich kann die Pumpe 18 zumindest optional dazu eingerichtet sein, Flüssigkeit aus dem Behandlungsgefäß 16 in das Vorratsgefäß 17 zurück zu pumpen, wie Pfeile 22, 23 andeuten. Die Förderrichtung und gegebenenfalls auch die Förderrate, mit der die Pumpe 18 arbeitet, legt die Steuerung 19 fest, wie durch einen Pfeil 24 angedeutet ist.

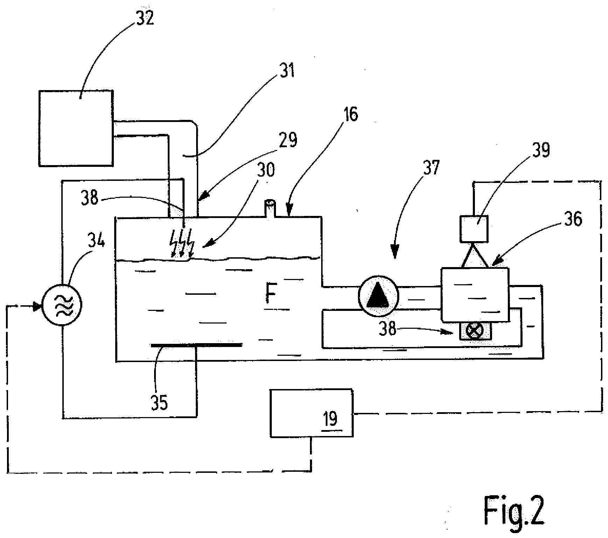

Das Behandlungsgefäß 16 kann ein Gefäß sein, in dem die zu behandelnde Flüssigkeit F, wie in Figur 2 skizzenhaft veranschaulicht ist, als kompakter Flüssigkeitskörper mit im Wesentlichen horizontaler Oberfläche gehalten ist. Als Behandlungsgefäß 16 kann jedoch jedes andere Gefäß dienen, in dem die Flüssigkeit mit einem Plasma in Berührung gebracht werden kann. Solche Gefäße sind beispielsweise Rieselgefäße, in denen ein Tröpfchenvorhang durch ein Plasma oder eine Flüssigkeitsschicht entlang eines Plasmas rieseln kann, Gefäße mit Zerstäubereinrichtung, die Flüssigkeit als Spray in ein Plasma einführen, Schleudervorrichtungen, die die Flüssigkeit als dünnen Film mit dem Plasma in Berührung bringen oder dergleichen mehr. Das Behandlungsgefäß 16 bildet mit einem darin erzeugten Plasma 30 (Figur 2) zusammen eine Plasmaapplikationseinrichtung 25.

Figur 1 veranschaulicht dazu in gestrichelt umrissenen Funktionsblöcken 26 - 28 verschiedene Mischvorrichtungen, von denen zumindest vorzugsweise wenigstens eine vorhanden ist und die dazu dienen, in dem Behandlungsgefäß entstandene reaktive Spezies homogen zu verteilen. Dazu kann ein Konvektor 26 dienen, der durch Einwirkung von Wärme oder Kälte auf die Flüssigkeit eine Konvektionsströmung erzeugt. Alternativ kann eine Rührvorrichtung 27 vorgesehen sein, die dazu dient, die Flüssigkeit F in dem Behandlungsgefäß 16 in Bewegung zu setzen. Ein Funktionsblock 28 veranschaulicht eine Gasverwirbelungsvorrichtung, um die Flüssigkeit in dem Behandlungsgefäß in innigen Kontakt mit dem Plasma 30 zu bringen. Jede der von den Funktionsblöcken 26, 27, 28 symbolisierte Einrichtung kann allein oder in Kombination mit einer oder mehreren der vorgenannten Einrichtungen in oder an dem Behandlungsgefäß 16 angeordnet sein.

Wie aus Figur 2 ersichtlich ist, ist dem Behandlungsgefäß 16 ein Plasma-Applikator 29 zugeordnet, der zur Erzeugung eines Plasmas 30 eingerichtet ist. Beispielsweise kann der Plasma-Applikator 29 ein Argon-Plasma-Applikator sein. Dieser weist einen Gaszuführungskanal 31 auf, über den ein Inertgas, z.B. Stickstoff, ein Edelgas, z.B. Argon oder Helium, oder ein anderes zur Plasmaerzeugung vorgesehenes Gas zum Beispiel ein reaktives Gas, z.B. ein sauerstoffhaltiges Gas, zugeführt wird. Der Gaskanal 31 ist an eine Gasquelle 32 angeschlossen. Diese kann, wie Figur 1 andeutet, steuerbar ausgebildet und an die Steuereinrichtung 19 angeschlossen sein, um von dieser hinsichtlich des Zeitpunkts der Gasabgabe und/oder hinsichtlich der Größe des Gasstroms gesteuert zu werden.

Der Plasma-Applikator weist außerdem eine vorzugsweise unisoliert ausgebildete, d.h., eine elektrisch leitende Oberfläche aufweisende Elektrode 33 auf, die von dem Gasstrom des Gaszuführungskanals 31 berührt oder umspült ist.

Die Elektrode ist an einen Pol eines Plasmagenerators 34 angeschlossen, dessen anderer Pol beispielsweise mit einer von der Flüssigkeit F umspülten Elektrode 35 verbunden ist. Der Plasmagenerator ist vorzugsweise ein HF-Generator, der zur Abgabe einer hochfrequenten Spannung und eines hochfrequenten Stromes eingerichtet ist. Der Generator ist vorzugsweise hinsichtlich der Größe der abgegebenen Spannung und/oder der abgegebenen Leistung und/oder des Stromes und/oder hinsichtlich der Kurvenform, der Modulation des Tastverhältnisses, des Crestfaktors oder anderer Parameter steuerbar. Der Plasmagenerator 34 kann dazu mit der Steuereinrichtung 19 verbunden und von dieser gesteuert werden, wie es Figur 1 veranschaulicht.

Die Einrichtung 10 weist außerdem eine Sensoreinrichtung 36 auf, die dazu dient und dazu eingerichtet ist, die Analyse der in der behandelten Flüssigkeit F gebildeten Spezies durchzuführen. Bei diesen Spezies handelt es sich um durch Plasmabehandlung der Flüssigkeit F gebildete Substanzen im weitesten Sinne, auch Ionen, Radikale, Molekülbruchstücke und dergleichen. Die Sensoreinrichtung 36 kann, wie Figur 2 schematisch veranschaulicht, außerhalb des Behandlungsgefäßes 16 angeordnet sein und beispielsweise über einen Kreislauf 37 mit Flüssigkeit F aus dem Behandlungsgefäß 16 versorgt werden.

In Figur 2 ist eine Sensoreinrichtung 36 zur optischen Absorptionsspektroskopie schematisch dargestellt. Dazu weist die Sensoreinrichtung 36 eine Lichtemissionseinrichtung 38 und eine Lichtaufnahmeeinrichtung 39 auf. Die Lichtemissionseinrichtung 38 ist beispielsweise dafür ausgelegt, ultraviolettes, sichtbares und/oder infrarotes Licht mit bekannter spektraler Zusammensetzung in die Flüssigkeit F abzugeben. Die Lichtaufnahmeeinrichtung 39 ist vorzugsweise dazu eingerichtet, die spektrale Zusammensetzung des ultravioletten, sichtbaren und/oder infraroten Lichts zu erfassen, das die Flüssigkeit F durchquert hat. Die Steuereinrichtung 19 ist dazu eingerichtet, aus dem Unterschied zwischen dem Spektrum des von der Lichtemissionseinrichtung 38 abgegebenen Lichts und des von der Lichtaufnahmeeinrichtung 39 aufgenommenen Lichts das Vorhandensein und die Konzentration ausgewählter Spezies zu ermitteln.

Anstelle der für die optische Absorptionsspektroskopie eingerichteten Sensoreinrichtung 36 kann auch jede andere Sensoreinrichtung vorgesehen sein, die dazu eingerichtet ist, das Vorhandensein und/oder die Konzentration einer oder mehrerer Spezies in der Flüssigkeit zu erfassen. Solche Sensoreinrichtungen können Emissions-Spektroskopie-Einrichtungen für ultraviolettes, sichtbares und/oder infrarotes Licht sein. Die Sensoreinrichtung kann auch eine Phosphoreszenz-Erfassungseinrichtung für ultraviolettes, sichtbares und/oder infrarotes Licht, insbesondere für nahes Infrarot sein. Auch kann die Sensoreinrichtung 36 eine Einrichtung zur pH-Messung, insbesondere eine Einstab-Messkette sein. Die Sensoreinrichtung 36 kann eine oder mehrere der vorgenannten Sensoreinrichtungen umfassen.

Die Steuereinrichtung 19 kann den Plasmagenerator 34 steuern, um die Konzentration und/oder Zusammensetzung verschiedener Spezies in der Flüssigkeit F zu steuern. Beispielsweise kann die Steuereinrichtung 19 den von dem Plasmagenerator 34 abgegebenen Strom und/oder die abgegebene Spannung hinsichtlich Leistung, Größe, Wellenform, Crestfaktor, Frequenz, Modulation und dergleichen steuern, um damit das Plasma und dadurch auch die Erzeugung von Spezies zu steuern.

Das Behandlungsgefäß 16 kann über eine Pumpe 40 direkt mit dem Instrument 11 verbunden sein, um dieses mit plasmaaktivierter Flüssigkeit zu versorgen. Optional kann zwischen der Pumpe 40 und dem Instrument 11 auch ein Lagergefäß 41 vorgesehen sein, in dem plasmaaktivierte Flüssigkeit über einen festgelegten oder wählbaren Zeitraum lagerbar ist.

Die Sensoreinrichtung 36 kann zusätzlich oder alternativ zu dem Kreislauf 37 mit dem Lagergefäß 41 verbunden sein. Ebenso kann das Lagergefäß 41 mit einer eigenen Sensoreinrichtung verbunden sein, die dann ihrerseits mit der Steuereinrichtung 19 verbunden ist. Durch Steuerung der Pumpe 40 und/der einer nicht weiter veranschaulichten zwischen dem Lagergefäß 41 und dem Instrument 11 angeordneten Pumpe kann die Steuereinrichtung 19 eine Lagerzeit für die plasmabehandelte Flüssigkeit F vorgeben. Nachdem sich nach einer Plasmabehandlung einer Flüssigkeit F die Konzentration der in der plasmabehandelten Flüssigkeit erzeugten Spezies mit unterschiedlicher Rate abnimmt, kann die Steuereinrichtung 19 durch Festlegung einer definierten Lagerzeit gegebenenfalls unter Kontrolle der Sensoreinrichtung 36, plasmabehandelte Flüssigkeit F abgeben, in der beispielsweise kurzlebige Spezies weitgehend verschwunden, langlebige Spezies jedoch in hoher Konzentration enthalten sind. Andererseits kann, wenn der Behandlungswunsch vor allem kurzlebige Spezies umfasst, mittels der Sensoreinrichtung 36 erfasst werden, ob diese in ausreichender Konzentration erzeugt sind, um sie dem Instrument 11 sofort zuzuführen. Gegebenenfalls kann dazu vorgesehen sein, Flüssigkeit aus dem Lagergefäß 41 zur Nachaktivierung dem Behandlungsgefäß 16 zuzuführen. Dies ist in Figur 1 durch die Doppelpfeile 42, 43 angedeutet.

Die insoweit beschriebene Einrichtung 10 und das Instrument 11 arbeiten wie folgt:
Über einen Einfüllstutzen 44 (Figur 1) wird das Vorratsgefäß 17 zunächst mit einer zu aktivierenden Flüssigkeit, beispielsweise Natrium-Chlorid-Lösung gefüllt. Die Steuereinrichtung 19 aktiviert nun die Pumpe 18 und führ Flüssigkeit F in das Behandlungsgefäß 16, z.B. indem sie das Behandlungsgefäß 16 mit der Flüssigkeit F füllt. Außerdem aktiviert die Steuereinrichtung 19 den Plasmagenerator 34 sowie gegebenenfalls die Gasquelle 32, sodass der Plasma-Applikator 29 nun ein auf die Flüssigkeit F einwirkendes Plasma 30 (Figur 2) erzeugt. Fortwährend oder in diskreten Zeitabständen wird die Sensoreinrichtung 36 aktiv, um die Qualität der Flüssigkeit F, d.h. deren Plasmaaktivierung zu erfassen. Die Qualität der Plasmaaktivierung bemisst sich dabei insbesondere nach der Konzentration ausgewählter Spezies wie beispielsweise dem Gehalt von Hydroniumionen, Hydroxidionen, Wasserstoffperoxid, Nitrit, Nitrat, Peroxynitrit, Singulett-Sauerstoff, Ozon, Hydroperoxyd, Sauerstoff, Superoxidradikalionen, Hydroxylradikalen und/oder Hydroperoxylradikalen. Zur Erfassung solcher Spezies ist die Sensoreinrichtung 36 als sogenannte pH-Einstabmesskette, als Spektroskop für emittiertes Licht (IR, sichtbares und/oder UV), als Absorptionsspektroskop (wie in Figur 2 veranschaulicht), als Phosphoreszenzsensor, beispielsweise für Strahlung der Wellenlänge von 1275 nm zur Messung von Singulett-sauerstoff oder als Elektronenspinresonanzspektroskop ausgebildet. Die Sensoreinrichtung 36 kann auch mehrere der genannten Messeinrichtungen umfassen.

Anhand der von der Sensoreinrichtung ermittelten Konzentrationen ausgewählter Spezies kann die Steuereinrichtung 19 die Plasmabehandlungsdauer der Flüssigkeit festlegen, verlängern oder verkürzen, die Parameter des von dem Plasmagenerator 34 abgegebenen Stroms oder der Spannung regulieren und/oder den Gasfluss der Gasquelle 32 beeinflussen. Zusätzlich oder alternativ kann die Steuereinrichtung 19 die Lagerzeit der plasmabehandelten Flüssigkeit in dem Lagergefäß 41 festlegen oder begrenzen und/oder eine oder mehrere der Einrichtungen 26 bis 28 steuern.

Die Steuereinrichtung 19 kann darauf eingerichtet sein, die Qualität der Flüssigkeit F hinsichtlich eines oder mehrerer der nachfolgend genannten Zusammenhänge zu beeinflussen:
- Beeinflussung der Effektstärke des Plasmas, d.h. der im Plasma umgesetzten elektrischen Leistung oder sonstiger Parameter, in Abhängigkeit vom gewünschten pH-Wert. Mit einer höheren Leistung wird ein niedrigerer pH-Wert erzielt.
- Festlegung der Einwirkungsdauer des Plasmas auf die Flüssigkeit in Abhängigkeit vom gewünschten pH-Wert. Mit einer höheren Einwirkungsdauer wird ein niedrigerer pH-Wert erzielt.
- Festlegung der Effektstärke des Plasmas, d.h. der im Plasma umgesetzten elektrischen Leistung oder sonstiger Parameter, in Abhängigkeit vom gewünschten Verhältnis von Wasserstoff zu Nitrat.
- Auswahl der zu behandelnden Flüssigkeit in Abhängigkeit vom gewünschten Verhältnis von Wasserstoff zu Nitrat. Die zur Auswahl bereit gehaltenen Flüssigkeiten können z.B. NaCl-Lösung und Phosphat-gepufferte-NaCl-Lösung sein.

Die Steuereinrichtung 19 kann zur Erzielung einer gewünschten Qualität der Flüssigkeit F, d.h. einer gewünschten Zusammensetzung der enthaltenen Spezies, den oder die von der Sensoreinrichtung 36 erfassten chemischen und/oder physikalischen Parameter der behandelten Flüssigkeit F dazu nutzen, um die Arbeitsweise der Plasmaapplikationseinrichtung 25 oder des Plasmagenerators 34 zu steuern. Bei der Steuerung des Plasmagenerators wird wenigstens ein Parameter des von ihm abgegebenen Stroms und/oder der von ihm abgegebenen Spannung beeinflusst.

Mit der Erfindung wird eine Einrichtung 10 zur Erzeugung plasmaaktivierter Flüssigkeit F mit definierten Eigenschaften bereitgestellt. Die Einrichtung 10 umfasst eine mit einem Plasma-Applikator 29 versehene Plasmaapplikationseinrichtung 25, in der eine Flüssigkeit F mit einem Gas-Plasma 30 in Berührung gebracht wird. Eine Sensoreinrichtung 36 dient der Analyse der Zusammensetzung der plasmabehandelten Flüssigkeit F wenigstens hinsichtlich einer durch die Plasmabehandlung erzeugten Spezies. Anhand der von der Sensoreinrichtung 36 erfassten Konzentration einer oder mehrerer Spezies können die Behandlungsparameter der Flüssigkeit F in der Plasmaapplikationseinrichtung 25 eingestellt oder verändert werden. Damit gelingt die Bereitstellung einer plasmabehandelten Flüssigkeit F mit definierten Eigenschaften zur Behandlung eines Patienten.

### Bezugszeichen:

- 10: Einrichtung zur Erzeugung plasmaaktivierter Flüssigkeit
- 11: Instrument
- 12 - 15: Funktionsblöcke zur Symbolisierung einer Behandlung mit plasmaaktivierter Flüssigkeit
- 16: Behandlungsgefäß
- 17: Vorratsgefäß
- 18: Pumpe
- 19: Steuereinrichtung
- 20, 21: Pfeile zur Pumprichtung der Pumpe 18
- 22, 23: Pfeile zur optionalen Pumprichtung der Pumpe 18
- 24: Pfeil bezüglich Steuerung der Pumpe 18
- 25: Plasmaapplikationseinrichtung
- 26: Funktionsblock Konvektor
- 27: Funktionsblock Rührvorrichtung
- 28: Funktionsblock Gasverwirbelungseinrichtung
- 29: Plasma-Applikator
- 30: Plasma
- 31: Gaszuführungskanal
- 32: Gasquelle
- 33: Elektrode
- 34: Plasmagenerator
- 35: Elektrode
- 36: Sensoreinrichtung
- 37: Kreislauf
- 38: Lichtemissionseinrichtung
- 39: Lichtaufnahmeeinrichtung
- 40: Pumpe
- 41: Lagergefäß
- 42, 43: Doppelpfeile
- 44: Einfüllstutzen

## Patentansprüche

1. Einrichtung (10) zur Versorgung eines medizinischen Instruments (11) mit einer plasmaaktivierten Flüssigkeit,
mit einem Plasmagenerator (34) zur Bereitstellung eines Plasmas (30),
mit einer Plasmaapplikationseinrichtung (25), in der eine Flüssigkeit (F) mit dem Plasma (30) in Kontakt überführbar ist und die einen Ausgang aufweist, um Flüssigkeit aus der Plasmaapplikationseinrichtung (25) auszuleiten und dem Instrument (11) zuzuleiten,
mit einer Sensoreinrichtung (36) zur Erfassung wenigstens eines chemischen oder physikalischen Parameters der Flüssigkeit (F) während und/oder nach der Plasmaexposition in der Plasmaapplikationseinrichtung (25).

2. Einrichtung nach Anspruch 1, **dadurch gekennzeichnet, dass** die Plasmaapplikationseinrichtung (25) einen Plasma-Applikator (29) mit einem Gaskanal (31) und wenigstens einer mit Gas aus dem Gaskanal in Berührung stehende Elektrode (33) sowie eine mit der Flüssigkeit in elektrischem Kontakt stehende Elektrode (35) aufweist, die beide an einen Plasmagenerator (34) angeschlossen sind.

3. Einrichtung nach einem der vorstehenden Ansprüche, **dadurch gekennzeichnet, dass** ein Ausgang der Plasmaapplikationseinrichtung (25) an ein Vorratsgefäß (41) angeschlossen ist, das mit dem Instrument (11) verbindbar ist.

4. Einrichtung nach Anspruch 3, **dadurch gekennzeichnet, dass** die Sensoreinrichtung (36) an wenigstens ein Steuerorgan angeschlossen ist, um die Verweilzeit der Flüssigkeit (F) in der Plasmaapplikationseinrichtung (25) zu steuern.

5. Einrichtung nach einem der vorstehenden Ansprüche, **dadurch gekennzeichnet, dass** der Plasmagenerator (34) hinsichtlich der von ihm abgegebenen Spannung, des Stromes, der Leistung, des Crest-Faktors oder der Wellenform des abgegebenen Stroms oder der abgegebenen Spannung steuerbar ist.

6. Einrichtung nach Anspruch 5, **dadurch gekennzeichnet, dass** die Sensoreinrichtung (36) an den Plasmagenerator (34) angeschlossen ist, um diesen in Abhängigkeit von dem erfassten Parameter zu steuern.

7. Einrichtung nach Anspruch 2, **dadurch gekennzeichnet, dass** der Gaskanal (31) an eine Gasquelle (32) angeschlossen ist, die hinsichtlich des Gasflusses steuerbar ist.

8. Einrichtung nach Anspruch 7, **dadurch gekennzeichnet, dass** die Sensoreinrichtung (19) an die Gasquelle (32) angeschlossen ist, um deren Gasfluss in Abhängigkeit von dem erfassten Parameter zu steuern.

9. Einrichtung nach einem der vorstehenden Ansprüche, **dadurch gekennzeichnet, dass** die Sensoreinrichtung (36) dazu eingerichtet ist, die Temperatur und/oder die Leitfähigkeit und/oder die Acidität (pH-Wert) und oder die chemische Zusammensetzung und/oder die Konzentration bestimmter chemischer Verbindungen zu erfassen.

10. Einrichtung nach einem der vorstehenden Ansprüche, **dadurch gekennzeichnet, dass** die Sensoreinrichtung (36) darauf eingerichtet ist, in der plasmaaktivierten Flüssigkeit die Konzentration von plasmaerzeugten Substanzen in Gestalt von Hydroniumionen(H₃O⁺) und/oder Hydroxidionen (OH⁻) und/oder Wasserstoffperoxid (H₂O₂) und/oder von Nitritionen (N0₂⁻) und/oder von NitratIonen (NO₃-) und/oder von Hydroxylradikalen (·OH) zu erfassen.

11. Einrichtung nach einem der vorstehenden Ansprüche, **dadurch gekennzeichnet, dass** die Sensoreinrichtung (36) darauf eingerichtet ist, in der plasmaaktivierten Flüssigkeit (F) die Konzentration von plasmaerzeugten Substanzen durch Spektroskopie beispielsweise Absorptionsspektroskopie elektromagnetischer Strahlung, insbesondere Licht, insbesondere sichtbarem Licht zu erfassen.

12. Einrichtung nach einem der vorstehenden Ansprüche, **dadurch gekennzeichnet, dass** die Sensoreinrichtung (36) darauf eingerichtet ist, in der plasmaaktivierten Flüssigkeit (F) die Konzentration von plasmaerzeugten Substanzen in Gestalt von Singulett Sauerstoff (¹O₂) und/oder Ozon (O₃) und/oder Sauerstoff (O₂) und/oder Superoxidradikalionen (O₂⁻) und/oder Hydroperoxylradikale (HOO·) und/oder von Peroxinitrit-Ionen (ONOO⁻) und/oder Stickoxiden wie beispielsweise Stickstoffmonoxid (NO) oder Stickstoffdioxid (NO₂) zu erfassen.

13. Einrichtung nach einem der vorstehenden Ansprüche,
**dadurch gekennzeichnet, dass** die Sensoreinrichtung (36) darauf eingerichtet ist, in der plasmaaktivierten Flüssigkeit (F) die Konzentration von plasmaerzeugten Substanzen mittels Phosphoreszenz oder Elektronenspinresonanz-Spektroskopie zu erfassen.

14. Verfahren zur Bereitstellung einer plasmaaktivierten Behandlungsflüssigkeit, bei dem:
mittels eines Plasmagenerators (34) ein Plasma (30) bereitgestellt wird,
in einer Plasmaapplikationseinrichtung (25) eine Flüssigkeit (F) mit dem Plasma (30) in Kontakt gebracht und dem Instrument (11) zugeleitet wird,
mittels einer Sensoreinrichtung (36) während und/oder nach der Plasmaexposition in der Plasmaapplikationseinrichtung (25) wenigstens ein chemischer oder physikalischer Parameter der Flüssigkeit (F) erfasst wird, der Betrieb des Plasmagenerators (34) und/oder die Verweilzeit der plasmabehandelten Flüssigkeit in einem Vorratsgefäß (41) von der Steuereinrichtung (19) zur Beeinflussung des Parameters gesteuert wird.
